# EUROPEAN PATENT SPECIFICATION

(11) **EP 2 908 902 B1**
(45) Date of publication and mention of the grant of the patent: **05.12.2018**
(21) Application number: 12784155.9
(22) Date of filing: 16.10.2012
(51) Int. Cl.: A61M 39/28, F16K 7/06, A61M 5/142, A61M 5/168, F16K 31/524, F16K 11/02

(54) **CONTROLLING FLOW IN A MEDICAL INJECTION SYSTEM**
FLUSSREGULIERUNG IN EINEM MEDIZINISCHEN INJEKTIONSSYSTEM
RÉGULATION DE FLUX DANS UN DISPOSITIF D'INJECTION MÉDICAL

(43) Date of publication of application: 26.08.2015
(73) Proprietor: Acist Medical Systems, Inc., Eden Prairie, MN 55344 (US)
(72) Inventor: RUSSELL, John, Cologne, MN 55322 (US); COOLIDGE, Troy, Marshall, Victoria, MN 55386 (US)
(74) Representative: Pistolesi, Roberto
(86) International application number: PCT/US2012/060380
(87) International publication number: WO 2014/062160

(56) References cited:
- WO-A1-01/55626
- GB-A- 2 274 326
- US-A- 3 299 904
- US-A- 3 802 463
- US-A- 3 813 077
- US-A- 5 113 906
- US-A1- 2005 255 426

## Description

### TECHNICAL FIELD

The present disclosure pertains to medical injection systems and more particularly to apparatus and methods for controlling flow therein.

### BACKGROUND

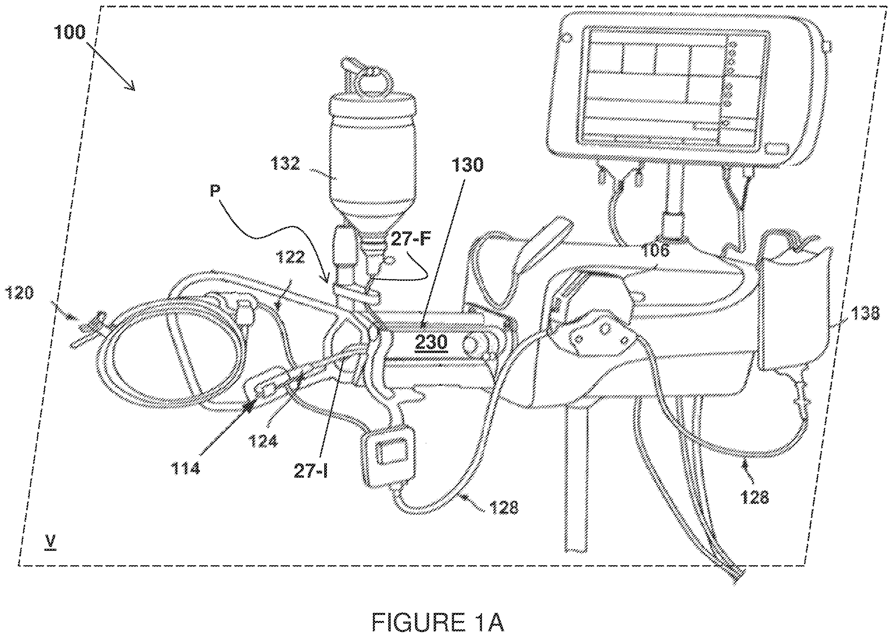

Figure 1A is a perspective view of an exemplary medical injection system 100 (the ACIST CVᵢ® system) for delivering a contrast agent into a patient's vascular system for medical imaging. Figure 1A illustrates a first fluid reservoir 132 for supplying a syringe-type positive displacement pump of a pressurizing unit 130, via a fill tubing line 27-F, and an injection tubing line 27-I coupled to unit 130 for injection of, for example, a radiopaque contrast agent, into a patient's vascular system via an inserted catheter (not shown), for example, coupled to a patient tubing line 122 at a connector 120 thereof. Figure 1A further illustrates a second fluid reservoir 138 from which a diluent, such as saline, is drawn by a peristaltic pump 106 through yet another tubing line 128 that feeds into tubing line 122. A manifold valve 124 and associated sensor 114 control the flow of fluids into tubing line 122, from pressurizing unit 130 and from tubing line 128.

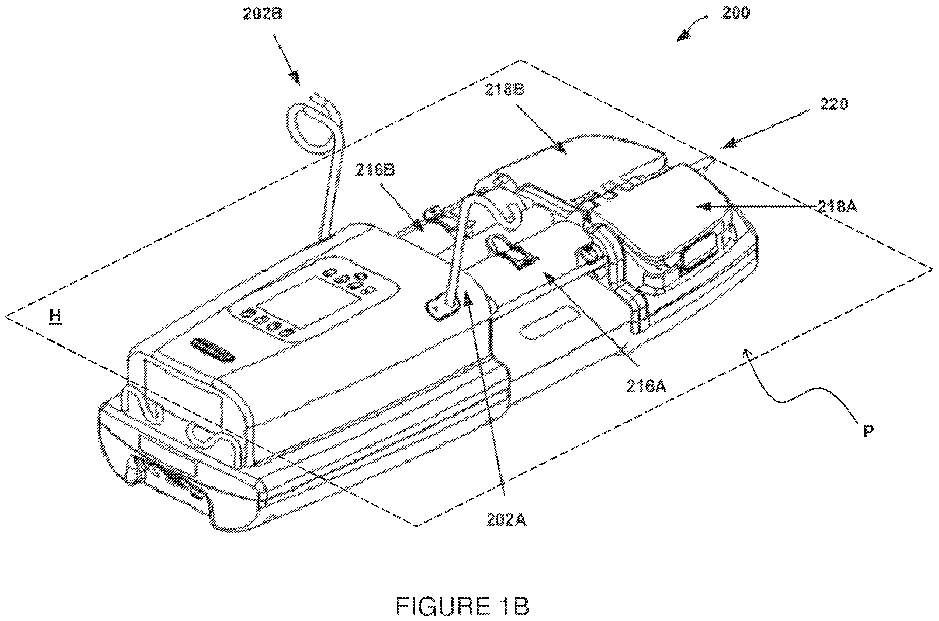

Although not shown in Figure 1, the syringe-type positive displacement pump of unit 130 includes a fill port and an injection port to which tubing lines 27-F and 27-I, respectively are coupled; a check valve (not shown) in line 27-F and valve 124 in line 27-I are employed to control flow through tubing lines 27-F and 27-I. For example, when the pump of unit 130 is activated to draw in fluid from reservoir 132, flow from tubing lines 122 and 128 through injection tubing line 27-I is blocked by valve 124; and, after the pump is filled, when the pump is activated to pressurize the fluid therein, valve 124 is opened to allow flow of the pressurized fluid through line 27-I and into line 122, while flow through fill tubing line 27-F is blocked by the aforementioned check valve. A variety of alternative valve mechanisms for controlling the flow through tubing lines of medical injection systems, such as tubing lines 27-F and 27-I of injection system 100, are known in the art. For example, a suitable pinch valve apparatus is described in commonly-assigned United States Patent 8,152,780. Yet, there is still a need for improved valve apparatus that simplify and increase the efficiency of valve operation to control flow in medical injection systems.

US 5,113,906 discloses a multiple rotary control valve of the pinch type for use in conjunction with a steam sterilizing apparatus. The valve comprises at least two side by side cams, each having cylindrical and flat portions for respectively pressing and releasing, in the proper sequence, flexible tubes conducting the fluids intervening in the sterilizing process.

US 2005/255426 discloses a switching device for an irrigation line. It includes two flexible supply tubes, each connecting a flask containing an irrigation fluid to a handpiece via a peristaltic pump. The device includes at least one compression element and control members which can apply the compression element alternatively to each of the flexible supply tubes in such a way as to squeeze and block the tube.

Other similar solutions are disclosed in US 3,299,904; US 3,813,077; US 3,802,463; GB 2,274,326 and WO 01/55626.

### SUMMARY

Apparatus and methods of the present invention are directed toward controlling flow through one or more tubing lines of a medical injection system. According to some methods, which may be employed by an injection system that includes fill and injection tubing lines coupled a pump, the two tubing lines are positioned in a valve apparatus such that a first of the two lines is located between a pinching member of the apparatus and a spring-loaded anvil of the apparatus, and a second of the two lines is located between the pinching member and a second spring-loaded anvil of the apparatus; subsequently, the pinching member is moved from a first position, at which the pinching member compresses the first tubing line against the first spring-loaded anvil, to a second position, at which the pinching member compresses the second tubing line against the second spring-loaded anvil, and vice versa. When the pinching member compresses the first tubing line, flow therethrough is blocked while the pump draws fluid in through the second tubing line; and, when the pinching member compresses the second tubing line, flow therethrough is blocked while the pump injects the fluid out through the first tubing line, for example, at an injection pressure of up to approximately 1200 psi, according to some embodiments and methods. In alternative injection systems, a single tubing line may be positioned in a similar valve assembly that includes only one spring-loaded anvil in conjunction with the pinching member.

According to some preferred embodiments, the pinching member is coupled to a rotatable shaft, for example, driven by a motor that moves the pinching member about a central axis of the shaft between the first and second positions, and
through a neutral position therebetween (where the pinching member may be located when the tubing lines are initially positioned in the valve apparatus). Furthermore, the pinching member is preferably rotatable about an auxiliary axis, which is eccentric, or offset from the central axis of the shaft, so that pinching member rolls into and out of contact with the tubing lines when moved back and forth between the first and second positions. A spring member of each of the spring-loaded anvils may be in the form of an elongate flexure that has a fixed end and a floating end, to which an anvil block, on which the corresponding anvil is formed, is attached. According to some preferred methods and embodiments, each spring-member is pre-loaded, for example, prior to moving the pinching member to compress one of the tubing lines in the valve apparatus. Furthermore, a minimum gap between each pre-loaded spring-loaded anvil and the pinching member in one or both of the first and second positions is preferably established for an approximate 20% squeeze reduction of the wall thickness of the corresponding tubing line minus a maximum in-service deflection of the corresponding pre-loaded spring member, which in-service deflection is preferably established, for a given spring constant of the pre-loaded spring member, to provide a pinching force of greater than approximately 45 pounds, with a variability within approximately 15% of nominal, over the range of in-service deflection.

### BRIEF DESCRIPTION OF THE DRAWINGS

The following drawings are illustrative of particular methods and embodiments of the present disclosure and, therefore, do not limit the scope of the invention. The drawings are not to scale (unless so stated) and are intended for use in conjunction with the explanations in the following detailed description. Methods and embodiments will hereinafter be described in conjunction with the appended drawings, wherein like numerals denote like elements, and:
Figures 1A-B are perspective views of at least portions of exemplary medical injection systems that may employ embodiments of the present invention;
Figure 2A is a plan view of fill and injection tubing lines that extend from a positive displacement pump and through a valve apparatus, according to some embodiments and methods of the present invention;
Figure 2B is an enlarged plan view of the apparatus of Figure 2A, according to some embodiments;
Figure 3 is a perspective view of the valve apparatus, according to some embodiments;
Figure 4A is a longitudinal cross-section view through section line 4-4 of Figure 2B, according to some embodiments;
Figure 4B is a schematic representation of an exemplary valve apparatus in which schematic spring elements are employed;
Figure 5A is a chart defining torque output of a suitable stepping motor, according to some embodiments; and
Figure 5B is another schematic for reference in conjunction with description of the exemplary valve apparatus.

### DETAILED DESCRIPTION

The following detailed description is exemplary in nature and is not intended to limit the scope, applicability, or configuration of the invention in any way. Rather, the following description provides practical illustrations for implementing exemplary methods and embodiments. Examples of constructions, materials and dimensions are provided for selected elements, and all other elements employ that which is known to those of skill in the field of the invention. Those skilled in the art will recognize that many of the examples provided have suitable alternatives that can be utilized.

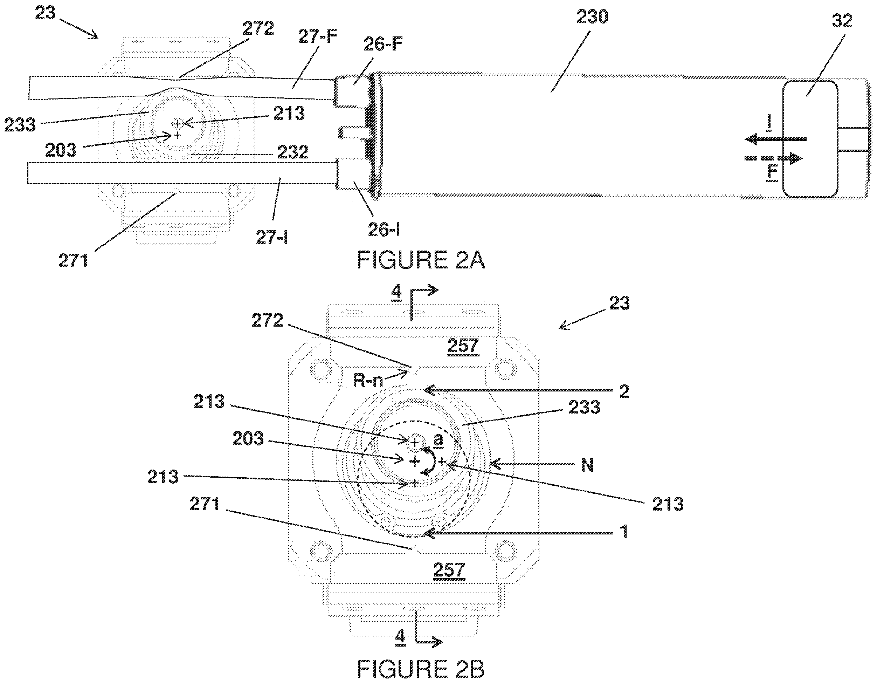

Figure 2A is a plan view of a portion of fill tubing line 27-F and a portion of injection tubing line 27-I, both of which extend from a positive displacement pump 230 and through a valve apparatus 23, according to some embodiments and methods of the present invention. Fill tubing line 27-F is shown coupled to a fill port 26-F of pump 230 and injection line 27-I to an injection port 26-I of pump 230. With reference back to Figure 1A, it may be appreciated that pump 230 may be employed by, and integrated into pressurizing unit 130 of system 100, and valve apparatus 23 may be employed in system 100, to take over for the aforementioned check valve and manifold valve 124 by controlling flow through lines 27-F and 27-I. Figure 2A illustrates valve apparatus 23 including a pinching member 233, a first spring-loaded anvil 271, and a second spring-loaded anvil 272; injection tubing line 27-I is shown extending between first anvil 271 and pinching member 233, and fill tubing line 27-F between second anvil 272 and pinching member 233. Each of tubing lines 27-F, 27-I may be formed from a nylon reinforced polyurethane or a flexible PVC material, both of which are known to those skilled in the art. In Figure 2A, pinching member 233 compresses fill tubing line 27-F against spring-loaded anvil 272 to block a flow of fluid therethrough from pump 230, when a plunger 32 of pump 230 moves per arrow I to pressurize and inject the fluid out through injection tubing line 27-I. Figure 2A further illustrates pinching member 233 being coupled to a shaft 232, which is preferably rotatable about a central axis 203 thereof, for example, back and forth according to double-headed arrow a of Figure 2B, to move member 233 between first and second positions 1, 2.

Figure 2B is an enlarged plan view of valve apparatus 23, wherein dashed lines indicate first position 1 of pinching member 233, prior to movement to second position 2, which is the same as that illustrated in Figure 2A. Figures 2A-B illustrate pinching member 233 being centered on an auxiliary axis 213, which is eccentric, or offset from central axis 203. With further reference to Figure 2B, a rotation of shaft 232, to move pinching member 233 between first position 1 and second position 2, through a neutral position N, is illustrated by three locations of auxiliary axis 213. It may be appreciated that, according to preferred methods of the present invention, pinching member 233 is initially located in neutral position N when pump 230 and tubing lines 27-F, 27-I are loaded into an injection system, for example, to increase the ease of positioning lines 27-F and 27-I between pinching member 233 and the corresponding spring-loaded anvil 271, 272 within valve apparatus 23. Then, once pump 230 and lines 27-F, 27-I are loaded, pinching member 233 is moved into first position 1 to block flow from line 27-I, before plunger 32 of pump 230 is moved, per arrow F (Figure 2A), to draw fluid in through tubing line 27-F. After plunger 32 draws a predetermined volume of fluid into pump 230, pinching member 233 is moved from first position 1 to second position 2, as illustrated in Figure 2A, to block flow through tubing line 27-F while pump 230 expels the fluid through line 27-I, for example, at an injection pressure of up to approximately 1200 psi.

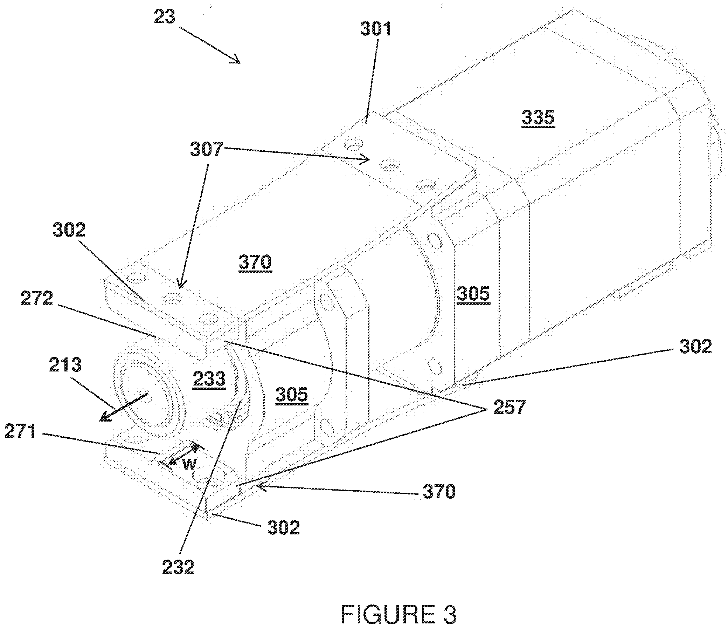

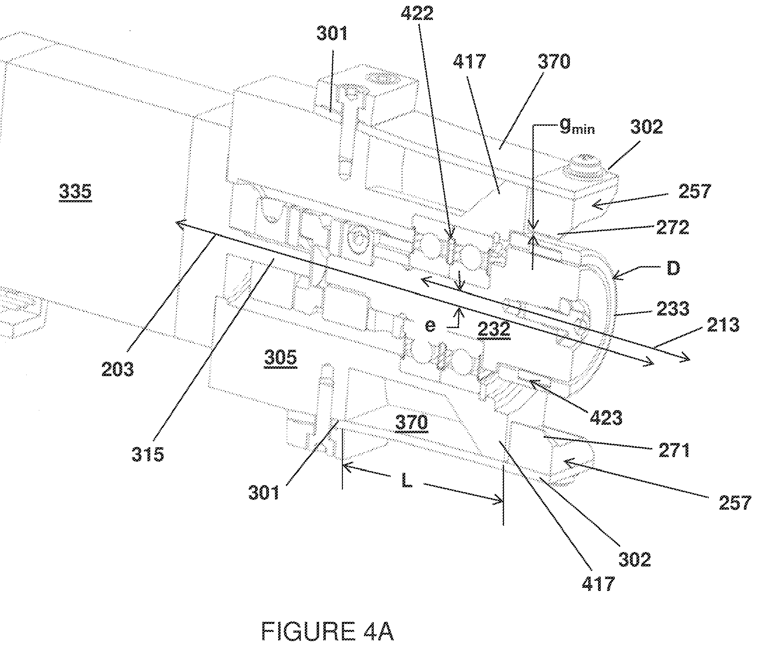

Figure 3 is a perspective view of valve apparatus 23, according to some embodiments. Figure 3 illustrates shaft 232 supported in a structure 305, for example, by a ball bearing assembly 422, which may be seen in the cross-section view of Figure 4A. Figure 4A is a longitudinal cross-section view through section line 4-4 of Figure 2B, according to some embodiments. Figure 4A further illustrates shaft 232 being coupled to a shaft 315 of a motor 335, for example, a stepping motor (i.e. Item # PK235PDA described at http://catalog.orientalmotor.com), which is contained in a fixed housing of valve apparatus 23. With reference back to Figure 1A, and as alluded to above, valve apparatus 23 may be employed by system 100, for example, by mounting apparatus 23 in the general vicinity indicated by reference letter P so that motor 335 sits to the rear of a generally vertical plane V, in which fill and injection ports 26-F, 26-I of pump 230 lie, and shaft 232 extends toward tubing lines 27-F, 27-I; thus, lines 27-F, 27-I may be positioned in valve apparatus 23 as illustrated in Figure 2A. Furthermore, with reference to Figure 1B, an injection system 200, which has an alternative configuration, may employ a pair of valve apparatus 23. Figure 1B illustrates system 200 including a pair of sleeves 216A, 216B, each of which may house a pump like pump 230 (Figure 2A), such that a set of tubing lines 27-F, 27-I, which are routed from each pump within a corresponding compartment 218A, 218B, lie in a generally horizontal plane H. According to the illustrated embodiment, one of the two pumps may pump saline, for example, filled form a reservoir mounted on a fixture 202A, and another of the pumps may pump a contrast agent, for example, filled from a reservoir mounted on a fixture 202B; a guide 220 is shown extending from compartments 218A, 218B for connecting the saline and contrast injection lines to a patient line. With further reference to Figure 1B, motor 335 of each valve apparatus 23 employed by system 200 may be mounted in the general vicinity indicated by reference letter P, below plane H, so that shaft 232 of each extends toward the corresponding set of tubing lines 27-F, 27-I; thus, each set of tubing lines may be positioned in the corresponding valve apparatus 23 as illustrated in Figure 2A.

Motor 335 of valve apparatus 23, when actuated (i.e. either user-actuated, or actuated in response to a trigger signal from a sensor coupled to pump 230), drives shaft 232 with sufficient torque to force pinching member 233, either into position 1, to compress tubing line 27-I against spring-loaded anvil 271, or into position 2, to compress tubing line 27-F against spring-loaded anvil 272, and thereby effectively block flow through lines 27-I and 27-F, in succession. As was generally described above, once tubing lines 27-I, 27-F are positioned in valve assembly 23, motor 335 may be actuated to move pinching member 233 to first position 1, at which member 233 compresses tubing line 27-I against anvil 271, while pump 230 draws in fluid from a reservoir, for example, reservoir 132 of Figure 1, through tubing line 27-F; then, once pump 230 is filled, motor is actuated to move pinching member 233 to second position 2 in order to compress tubing line 27-F while pump injects the fluid through tubing line 27-I. Over the course of one or more imaging procedures, pump 230 may need to be filled as many as approximately 5 times up to approximately 25 times; thus pinching member 233 is repeatedly moved between first and second positions 1, 2 enough times to replenish the volume of pump 230 and thereby support the required number of injections. According to some preferred methods, motor 335 drives shaft back and forth through neutral position N, approximately 180 degrees about central axis 203, to move pinching member 233, between first and second positions 1, 2; alternatively, motor 335 may drive shaft 232 360 degrees around central axis 203 to move pinching member 233 between first and second positions 1, 2. The stepping motor preferably includes an encoder to monitor the steps of the rotation of shaft 232 in order to keep track of, and confirm the position of pinching member 233 during system operation.

Figure 3 further illustrates each of anvils 271, 272 spring-loaded by virtue of attachment to a corresponding spring member, each of which is in the form of an elongate flexure 370. Figure 3 illustrates a first end 301 of each flexure 370 attached to support structure 305, and each of anvils 271, 272 being formed on a corresponding anvil block 257, which is fastened to a floating second end 302 of the corresponding flexure 370. Reference numeral 307 designates locations for exemplary fasteners, some of which may be seen in Figure 4A. Each of anvils 271, 272 preferably extends over a width w that is greater than or equal to an outer diameter of each of tubing lines 27-F, 27-I, and each may have a radius R-n of between approximately 0.06 inch (1.5 mm) and approximately 0.08 inch (2 mm), for example, to effectively pinch tubing lines 27-I, 27-F without damaging the walls thereof by applying undue stress. Anvil blocks 257 are preferably formed from stainless steel.

With further reference to Figures 2B and 4A, for a given wall thickness and material properties of the tubes forming tubing lines 27-F, 27-I, a minimum gap gₘᵢₙ is established between pinching member 233 and each of anvils 271, 272. (gₘᵢₙ is shown between second anvil 272 and member 233 in second position 2.) When tubing lines, for example, lines 27-I and 27-F, are positioned within valve apparatus 23, for example as illustrated in Figure 2A, the spring loading provided by flexures 370 establishes a relatively constant pinching pressure between each of anvils 271, 272 and pinching member 233 in the first and second positions 1, 2, respectively, for example, across a standard tolerance range wall thickness for the tubing forming each of tubing lines 27-I, 27-F. Furthermore, the spring loading of anvils 271, 272 maintains the relatively constant pinching pressure if the stiffness of either tubing line 27-I, 27-F creeps/declines in response to multiple pinch cycles, for example, up to as many as approximately 25 cycles, which may take place over the course of an imaging procedure, or a day of multiple imaging procedures, as described above.

With reference back to Figure 4A, according to some preferred methods and embodiments, flexures 370 are pre-loaded to establish a desired pinching force applied by pinching member 233, and to reduce an amplitude of cyclic loading seen by each flexure 370 over the life of valve apparatus 23, for example, corresponding to up to approximately 60,000 pinch cycles. Figure 4A illustrates pre-load supports 417, each of which is preferably formed in support structure 305; each support 417 is located adjacent anvil block 257 at floating second end 302 of the corresponding elongate flexure 370.

Figure 4A further illustrates pinching member 233 supported by a needle bearing assembly 423, such that member 233 is rotatable around auxiliary axis 213. Such support is preferred to allow pinching member 233 to roll into and out of contact with tubing lines 27-F, 27-I, thereby facilitating the movement of pinching member into, and out from first and second positions 1, 2 without displacing the corresponding tubing line 27-F, 27-I along a direction of its longitudinal axis, and/or without undue wear on tubing lines 27-F, 27-I.

### EXAMPLE

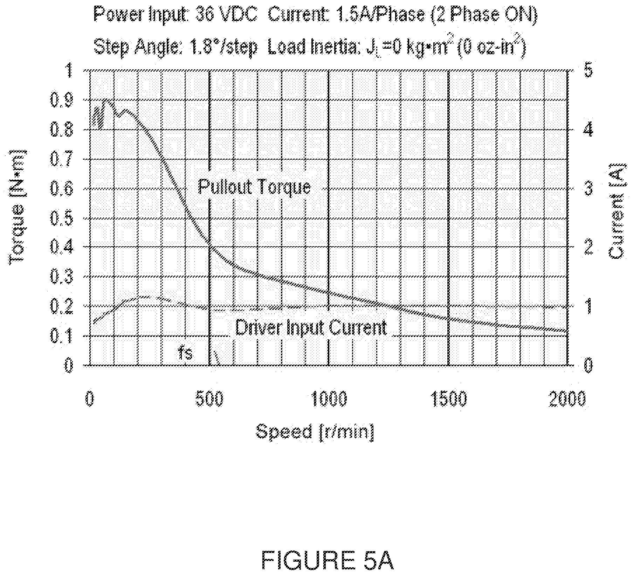

According to an exemplary embodiment, each tubing line 27-I, 27-F is formed by a nylon-reinforced polyurethane tube having a nominal outer diameter of approximately 0.188 inch (4.78 mm) and a nominal wall thickness t of approximately 0.05 inch (1.27 mm). Pinching member 233 and each spring-loaded anvil 271, 272 are suitably dimensioned, supported and arranged in valve apparatus 23 so that member 233 and each anvil 271, 272 apply a sufficient pinching force to each of the exemplary tubing lines 27-I, 27-F, when each tubing line is positioned in valve apparatus 23 (i.e. per Figure 2A), and when member 233 is in the first and second positions 1, 2, respectively. The sufficient pinching force, which prevents flow through the exemplary tubing lines at a maximum injection pressure of approximately 1200 psi has been found to be approximately 45 pounds, but, in order to assure a tubing failure (i.e. burst at approximately 1500 psi) prior to a cracking/leak through the pinching of fill tubing line 27-F, between pinching member 233 and spring-loaded anvil 272, at injection pressures exceeding the 1200 psi, a safety margin of 10 pounds brings the pinching force to approximately 55 pounds for the exemplary embodiment. Thus, prevention of backflow through fill tubing line 27-F is assured during injections through injection tubing line 27-I. Furthermore, in the exemplary embodiment, the aforementioned exemplary stepping motor (Item # PK235PDA; http://catalog.orientalmotor.com), which operates according to the torque curve shown in Figure 5A, applies sufficient torque, via shafts 315 and 232, to repeatedly move pinching member 233 from first position 1 to second position 2, and vice versa, without stalling, when the aforementioned tubes for each tubing line 27-I, 27-F are positioned in valve apparatus 23.

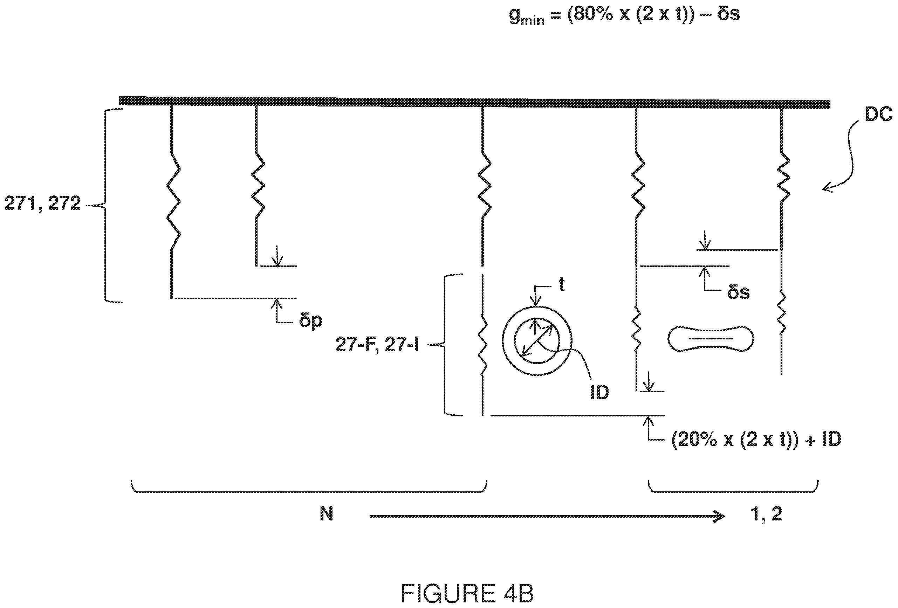

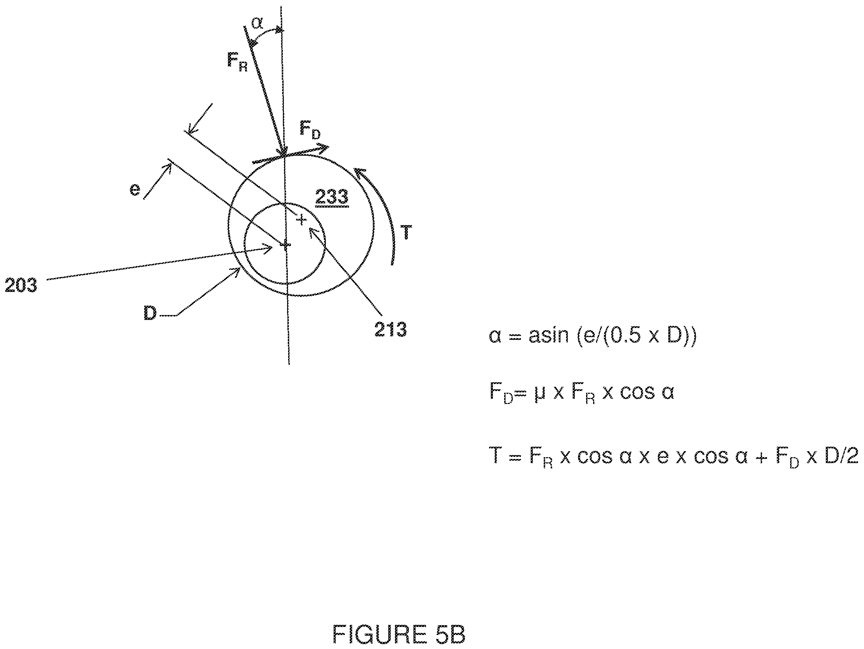

With reference to Figure 4A, each exemplary elongate flexure 370 is formed from a fully hardened stainless steel strip, which has a spring constant of approximately 439 lb/in; and each flexure 370 extends between the corresponding fixed end 301 and the corresponding anvil block 257, over a length L, which is approximately 1.42 inch. The minimum gap gₘᵢₙ, which is between first anvil 271 and pinching member 233, when member 233 is in first position 1(Figure 2B), and between second anvil 272 and pinching member 233, when member is in second position 2, as shown in Figure 4A, is set to approximately 0.05 inch (1.27 mm) for the exemplary embodiment. With reference to the schematic shown in Figure 4B, this minimum gap gₘᵢₙ is established according to a desired 20% squeeze reduction of the wall thickness t of each of tubing lines 27-I, 27-F and according to a maximum in-service deflection δs of spring-loaded anvils 271, 272, which is approximately 0.03 inch (0.76 mm). Figure 4B illustrates a deflection of tubing lines 27-F, 27-I equal to the 20% reduction plus an inner diameter ID of each tubing line. Figure 4B further illustrates a pre-load deflection δp, which, when established at approximately 0.09 inch for each spring-loaded anvil 271, 272, for example, by pre-load supports 417 abutting each exemplary flexure 370 (Figure 4A), assures that a radial force F_{R} (shown in the schematic of Figure 5B), applied to positioned tubing lines 27-I, 27-F by each of spring-loaded anvils 271, 272, with pinching member 233 positioned in the first and second positions 1, 2, respectively, is approximately maintained at the aforementioned 55 pound pinching force. It should be noted that Figure 4B illustrates spring-loaded anvils 271, 272 and tubing lines 27-F, 27-I as schematic spring elements transitioning in response to deflections imposed by pre-loading and by movement of pinching member 233 from the above-described neutral position N and into one of the above-described first and second positions 1, 2, wherein pinching member 233 is at top or bottom dead center DC when the maximum in-service deflection δs is achieved. According to preferred embodiments, the spring constant of flexures 370, minimum gap gₘᵢₙ and pre-load deflection δp determine a maximum in-service deflection δs, for the 20% squeeze reduction, that limits a variation in the 55 pound pinching force, for example, to within approximately 15% of nominal, throughout the range of in-service deflection δs (i.e. from initial compression of each tubing line 27-F, 27-I to top dead center DC).

Figure 5B is a schematic demonstrating how a diameter D of pinching member 233 and an offset e, in conjunction with radial force F_{R} and a drag force F_{D}, dictate the torque necessary to move member 233 into first and second positions 1, 2 (Figure 2B). The aforementioned pre-load deflection δp, along with a maximum rolling friction coefficient µ of approximately 0.005, for needle bearing assembly 423 with respect to a perimeter of pinching member 233, helps to limit the drag force F_{D} on pinching member 233 as member 233 rolls into contact with each of positioned tubing lines 27-F, 27-I. Furthermore, diameter D of pinching member 233, which is set to approximately 1.1 inch (28 mm), and offset e of auxiliary axis 213 from central axis 203, which is set to approximately 0.118 inch (3 mm), establish a maximum pressure angle α of approximately 12.4 degrees, which also contributes to limiting the drag force F_{D}. Equations for both the maximum pressure angle and the drag force are shown in Figure 5B, as is the equation governing the maximum torque T required to move pinching member 233 into the first and second positions 1, 2. Thus, according to the exemplary embodiment, the aforementioned stepping motor, performing according to the torque curve shown in Figure 5A, is able to repeatedly move pinching member 233 into the first and second positions 1, 2, at which the aforementioned pinching force is applied to each tubing line 27-I, 27-F, respectively. Furthermore, the motor may drive shaft 232 at a speed to move pinching member 233 from neutral position N to either of the first and second positions 1, 2, within a time of approximately 250 milliseconds. Preferably, the motor moves pinching member 233 back and forth, through 180 degrees, between the first and second positions 1, 2, as described above in conjunction with Figure 2B; but this exemplary embodiment does not rule out 360 degree movement of pinching member 233.

Finally, it should be noted that embodiments of the disclosed valve apparatus may be employed to control flow only through injection lines, for example, in systems that do not require pump filling and the associated fill tubing lines. In this context, the above-described embodiments may operate to control flow through two injection lines, successively, by moving back and forth between the two positions 1, 2, or may operate to control flow in a single injection line, for example, by only moving between the neutral position N and the first position 1.

## Claims

1. A medical injection system (100, 200) comprising a pump (230) having an inlet port (26-F) and an outlet port (26-1); a first tubing line (27-1) including a first end coupled to the outlet port (26-1) of the pump and a second end adapted for connection to a patient line (122) for injection of fluid from the pump; a second tubing line (27-F) including a first end coupled to the inlet port (26-F) of the pump and a second end adapted for connection to a fluid reservoir (132) for filling the pump with the fluid; and a valve apparatus (23) for controlling flow into and out from the pump, the valve apparatus comprising:
a rotatable shaft (232);
a pinching member (233) coupled to the rotatable shaft such that the pinching member moves around a central axis (203) of the shaft when the shaft is rotated;
a first anvil (271) attached to a first spring member and located on a first side of the shaft; and
a second anvil (272) attached to a second spring member and located on a second side of the shaft, the second side being generally opposite the first side;
wherein the first tubing line is positioned between the pinching member and the first anvil of the valve apparatus, and the second tubing line is positioned between the pinching member and the second anvil of the valve apparatus;
wherein rotation of the shaft moves the pinching member from a first position (1) to a second position (2);
the pinching member compresses the first tubing line against the first anvil at the first position; and
the pinching member compresses the second tubing line against the second anvil at the second position,
***characterized in that*** each of the first and second spring members of the valve apparatus comprises an elongate flexure (370), each flexure having a fixed first end (301) and a floating second end (302), each second end having the corresponding anvil attached thereto, wherein the valve apparatus further comprises a pair of pre-load supports (417), each pre-load support being located adjacent a corresponding floating second end of the corresponding flexure for pre-loading thereof and **in that** the pinching member (233) of the valve apparatus (23) is rotatably mounted about an auxiliary axis (213) that is offset from the central axis (203) of the shaft (232) so that the pinching member rolls into and out of contact with the first tubing line (27-I) and the second tubing line (27-F) when moved between the first and second positions.

2. The system (100, 200) of claim 1, wherein the valve apparatus (23) further comprises a fixed structure (305) supporting the shaft (232), the first fixed end (301) of each flexure (370) being coupled to the structure, and each pre-load support (417) being formed in the structure.

3. The system (100, 200) of claim 1, wherein:
a minimum gap between each anvil (271, 272) and the pinching member (233) in the corresponding position corresponds to an approximate 20% squeeze reduction of a wall thickness of the corresponding tubing line (27-I, 27-F) minus a maximum in-service deflection of each pre-loaded flexure (370); and
the maximum in-service deflection and a spring constant of each pre-loaded flexure provide a pinching force on each tubing line being compressed between the pinching member and the corresponding spring-loaded anvil of greater than approximately 200 N (45 pounds) that varies within approximately 15% of nominal over the range of the maximum in-service deflection.

4. The system (100, 200) of claim 1, wherein each anvil (271, 272) of the valve apparatus (23) has a radius (R-n) between approximately 1.5 mm (0.06 inch) and approximately 2 mm (0.08 inch).

5. The system of claim 1, wherein the pump (230) is configured to expel fluid through the first tubing line (27-I) at an injection pressure of up to approximately 8.27 MPa (1200 psi).

6. The system (100, 200) of claim 5, wherein the pump (230) is configured to expel fluid through the first tubing line (27-1) at an injection pressure of up to approximately 8.27 MPa (1200 psi) when the pinching member (233) is at the second position (2).

7. The system (100, 200) of claim 1, wherein the pump (230) is configured to fill with the fluid when the pinching member (233) is at the first position (1).

8. The system (100, 200) of claim 1, wherein pre-loading of the corresponding floating second end (302) of the corresponding flexure (370) comprises a pre-load deflection of 2.29 mm (0.09 inch) for the corresponding anvil (271, 272) attached thereto.

9. The system (100, 200) of claim 1, wherein the pinching member (233) further has a neutral position (N) between the first position (1) and the second position (2), and wherein when the pinching member (233) is moved from the first position to the second position the pinching member moves through the neutral position.

10. The system (100, 200) of claim 9, wherein the pinching member (233) compresses neither of the first tubing line (27-I) and the second tubing line (27-F) when at the neutral position (N).

## Patentansprüche

1. Medizinisches Injektions-System (100, 200), welches umfasst, eine Pumpe (230) mit einer Einlassöffnung (26-F) und einer Auslassöffnung (26-I); eine erste Schlauchleitung (27-I) deren erstes Ende mit der Auslassöffnung (26-I) der Pumpe gekoppelt ist und deren zweites Ende zur Verbindung mit einer zu einem Patienten führenden Leitung (122) zur Injektion von Fluid aus der Pumpe angepasst ist; eine zweite Schlauchleitung (27-F) deren erstes Ende mit der Einlassöffnung (26-F) der Pumpe gekoppelt ist und deren zweites Ende zur Verbindung mit einem Fluid-Reservoir (132) zum Befüllen der Pumpe mit dem Fluid angepasst ist; und eine Ventil-Vorrichtung (23) zur Steuerung des Stroms in und aus der Pumpe heraus, worin die Ventil-Vorrichtung umfasst:
eine drehbare Welle (232);
ein Drück-Element(233), das mit der drehbaren Welle derart gekoppelt ist, dass sich das Drück-Element um eine zentrale Achse (203) der Welle bewegt, wenn sich diese dreht;
einen ersten Amboss (271), der an einem erstes Federelement angebracht und an der ersten Seite der Welle angeordnet ist; und
einen zweiten Amboss (272), der an einem zweiten Federelement angebracht und an einer zweiten Seite der Welle angeordnet ist, worin die zweite Seite im Wesentlichen abgewandt zu der ersten Seite ist;
worin die erste Schlauchleitung zwischen dem Drück-Element und dem ersten Amboss der Ventil-Vorrichtung angeordnet ist, und worin die zweite Schlauchleitung zwischen dem Drück-Element und dem zweiten Amboss der Ventil-Vorrichtung angeordnet ist;
worin die Rotation der Welle das Drück-Element von einer ersten Position (1) in eine zweite Position (2) bewegt;
das Drück-Element die erste Schlauchleitung gegen den ersten Amboss an der ersten Position zusammendrückt; und
das Drück-Element die zweite Schlauchleitung gegen den zweiten Amboss an der zweiten Position zusammendrückt;
**dadurch gekennzeichnet, dass** jedes des ersten und zweiten Federelements der Ventil-Vorrichtung ein längliche Biegung (370) aufweist, worin jede Biegung ein fixiertes erstes Ende (301) und ein freies zweites Ende (302) aufweist, worin jedes zweite Ende den entsprechenden Amboss daran angebracht hat,
worin die Ventil-Vorrichtung weiter ein Paar Vorspannungsträger (417) aufweist, worin jeder Vorspannungsträger neben einem entsprechenden freien zweiten Ende der jeweiligen Biegung vorgesehen ist, um es vorzuspannen, und dass das Drück-Element(233) der Ventil-Vorrichtung (23) drehbar um eine Hilfsachse (213) angeordnet ist, die von einer zentralen Achse (203) der Welle (232) versetzt ist, so dass das Drück-Element, wenn es zwischen der ersten und zweiten Position bewegt wird, in und aus dem Kontakt mit der ersten Schlauchleitung (27-I) und der zweiten Schlauchleitung (27-F) rollt,.

2. System (100, 200) nach Anspruch 1, worin die Ventil-Vorrichtung (23) weiter einen fixierten Aufbau (305) umfasst, der die Welle (232) trägt, worin das erste fixierte Ende (301) einer jeden Biegung (370) mit dem Aufbau gekoppelt ist, und jeder vorgespante Träger (147) in dem Aufbau ausgebildet ist.

3. System (100, 200) nach Anspruch 1, worin:
ein minimaler Spalt zwischen jedem Amboss (271, 272) und dem Drück-Element(233) in der entsprechenden Position einer etwa 20%igen Druckreduzierung einer Wanddicke der jeweiligen Schlauchleitung (27-I, 27-F) minus einer maximalen Durchbiegung einer jeden vorgespannten Biegung (370) im Betrieb entspricht; und
die maximale Durchbiegung im Betrieb und eine Federkonstante einer jeden vorgespannten Biegung eine Druckkraft auf jede Schlauchleitung, die zwischen dem Drück-Element und dem jeweiligen Feder-vorgespannten Amboss komprimiert ist, von über etwa 200 N (45 Pfund) liefert, welche binnen etwa 15% des Nominalen über den Bereich der maximalen Durchbiegung im Betrieb variiert.

4. System (100, 200) nach Anspruch 1, worin jeder Amboss (271,272) der Ventil-Vorrichtung (23) einen Radius (R-n) zwischen etwa 1,5 mm (0,06 Inch) und etwa 2 mm (0,08 Inch) aufweist.

5. System nach Anspruch 1, worin die Pumpe (230) ausgestaltet ist, Fluid durch die erste Schlauchleitung (27-I) mit einem Injektionsdruck von bis zu etwa 8,27 MPa (1200 Psi) auszugeben.

6. System (100, 200) nach Anspruch 5, worin die Pumpe (230) ausgestaltet ist, Fluid durch die erste Schlauchleitung (27-I) bei einem Injektionsdruck von bis zu etwa 8,27 MPa (1200 Psi) auszugeben, wenn das Drück-Element(233) an der zweiten Position (2) ist.

7. System (100, 200) nach Anspruch 1, worin die Pumpe (230) ausgestaltet ist, mit dem Fluid gefüllt zu werden, wenn das Drück-Element(233) an der ersten Position (1) ist.

8. System (100, 200) nach Anspruch 1, worin die Vorspannung des jeweiligen freien zweiten Endes (302) der entsprechenden Biegung (370) eine Vorspannungs-Durchbiegung von 2,29 mm (0,09 Inch) für den jeweiligen daran angebrachten Amboss (271,272) umfasst.

9. System (100, 200) nach Anspruch 1, worin das Drück-Element (233) weiter eine neutrale Position (N) zwischen der ersten Position (1) und der zweiten Position (2) aufweist, und worin wenn das Drück-Element(233) von der ersten Position zu der zweiten Position bewegt wird, sich das Drück-Element durch die neutrale Position bewegt.

10. System (100, 200) nach Anspruch 9, worin das Drück-Element(233) weder die erste Schlauchleitung (27-I) noch die zweite Schlauchleitung (27-F) komprimiert, wenn es in der neutralen Position (N) ist.

## Revendications

1. Système d'injection médical (100, 200) comprenant une pompe (230) ayant un orifice d'entrée (26-F) et un orifice de sortie (26-1) ; une première ligne de tube (27-1) comprenant une première extrémité couplée à l'orifice de sortie (26-1) de la pompe et une seconde extrémité adaptée pour le raccordement à une ligne de patient (122) pour l'injection de fluide à partir de la pompe ; une seconde ligne de tube (27-F) comprenant une première extrémité couplée à l'orifice d'entrée (26-F) de la pompe et une seconde extrémité adaptée pour le raccordement à un réservoir de fluide (132) pour remplir la pompe avec le fluide ; et un appareil de valve (23) pour réguler l'écoulement à l'intérieur de et à l'extérieur de la pompe, l'appareil de valve comprenant :
un arbre rotatif (232) ;
un élément de pincement (233) couplé à l'arbre rotatif de sorte que l'élément de pincement se déplace autour d'un axe central (203) de l'arbre lorsque l'arbre est entraîné en rotation ;
une première enclume (271) fixée sur un premier élément de ressort et positionnée sur un premier côté de l'arbre ; et
une seconde enclume (272) fixée à un second élément de ressort et positionnée sur un second côté de l'arbre, le second côté étant généralement opposé au premier côté ;
dans lequel la première ligne de tube est positionnée entre l'élément de pincement et la première enclume de l'appareil de valve, et la seconde ligne de tube est positionnée entre l'élément de pincement et la seconde enclume de l'appareil de valve ;
dans lequel la rotation de l'arbre déplace l'élément de pincement d'une première position (1) à une seconde position (2) ;
l'élément de pincement comprime la première ligne de tube contre la première enclume dans la première position ; et
l'élément de pincement comprime la seconde ligne de tube contre la seconde enclume dans la seconde position ;
**caractérisé en ce que** :
chacun des premier et second éléments de ressort de l'appareil de valve comprend une courbure allongée (370), chaque courbure ayant une première extrémité fixe (301) et une seconde extrémité flottante (302), chaque seconde extrémité ayant l'enclume correspondante fixée à cette dernière,
dans lequel l'appareil de valve comprend en outre une paire de supports de pré-charge (417), chaque support de pré-charge étant positionné de manière adjacente à une seconde extrémité flottante correspondante de la courbure correspondante pour sa pré-charge et **en ce que** l'élément de pincement (233) de l'appareil de valve (23) est monté, de manière rotative, autour d'un axe auxiliaire (213) qui est décalé de l'axe central (203) de l'arbre (232) de sorte que l'élément de pincement roule en et hors de contact avec la première ligne de tube (27-1) et la seconde ligne de tube (27-F) lorsqu'il est déplacé entre les première et seconde positions.

2. Système (100, 200) selon la revendication 1, dans lequel l'appareil de valve (23) comprend en outre une structure fixe (305) supportant l'arbre (232), la première extrémité fixe (301) de chaque courbure (370) étant couplée à la structure, et chaque support de pré-charge (417) étant formé dans la structure.

3. Système (100, 200) selon la revendication 1, dans lequel :
un espace minimum entre chaque enclume (271, 272) et l'élément de pincement (233) dans la position correspondante correspond à une réduction de compression d'approximativement 20% d'une épaisseur de paroi de la ligne de tube (27-1, 27-F) correspondante moins une déflexion de service maximum de chaque courbure pré-chargée (370) ; et
la déflexion en service maximum et une constante de rappel de chaque courbure pré-chargée fournissent une force de pincement sur chaque ligne de tube qui est comprimée entre l'élément de pincement et l'enclume à ressort correspondante, supérieure à approximativement 200 N (45 livres) qui varie dans une plage d'approximativement 15% d'une valeur nominale sur la plage de la déflexion en service maximum.

4. Système (100, 200) selon la revendication 1, dans lequel chaque enclume (271, 272) de l'appareil de valve (23) a un rayon (R-n) compris entre approximativement 1,5 mm (0,06 pouce) et approximativement 2 mm (0,08 pouce).

5. Système selon la revendication 1, dans lequel la pompe (230) est configurée pour expulser le fluide par la première ligne de tube (27-1) à une pression d'injection jusqu'à approximativement 8,27 MPa (1200 psi).

6. Système (100, 200) selon la revendication 5, dans lequel la pompe (230) est configurée pour expulser le fluide par la première ligne de tube (27-1) à une pression d'injection jusqu'à approximativement 8,27 MPa (1200 psi) lorsque l'élément de pincement (233) est dans la seconde position (2).

7. Système (100, 200) selon la revendication 1, dans lequel la pompe (230) est configurée pour se remplir avec le fluide lorsque l'élément de pincement (233) est dans la première position (1).

8. Système (100, 200) selon la revendication 1, dans lequel la pré-charge de la seconde extrémité flottante (302) correspondante de la courbure (370) correspondante comprend une déflexion de pré-charge de 2,29 mm (0,09 pouce) pour l'enclume (271, 272) correspondante fixée à cette dernière.

9. Système (100, 200) selon la revendication 1, dans lequel l'élément de pincement (233) a en outre une position neutre (N) entre la première position (1) et la seconde position (2), et dans lequel lorsque l'élément de pincement (233) passe de la première position à la seconde position, l'élément de pincement se déplace en passant par la position neutre.

10. Système (100, 200) selon la revendication 9, dans lequel l'élément de pincement (233) ne comprend pas la première ligne de tube (27-1) ni la seconde ligne de tube (27-F) lorsqu'il est dans la position neutre (N).
